# FASCICULE DE BREVET EUROPEEN

(11) **EP 1 132 109 B1**
(45) Date de publication et mention de la délivrance du brevet: **18.07.2007**
(21) Numéro de dépôt: 01400601.9
(22) Date de dépôt: 07.03.2001
(51) Int. Cl.: A61N 1/365, A61N 1/368

(54) **Stimulateur implantable du type multisite comportant des moyens de mesure d'impédance intracardiaque**
Implantierbarer Mehrstellen-Stimulater mit Mitteln zum Messen der introkardialen Impedanz
Implantable multi-site stimulator with means for measuring intra-cardiac impedance

(30) Priorité: 07.03.2000 FR 0002879
(43) Date de publication de la demande: 12.09.2001
(73) Titulaire: ELA MEDICAL, F-92541 Montrouge (FR)
(72) Inventeur: Dal Molin, Renzo, 92320 Chatillon (FR)
(74) Mandataire: Dupuis-Latour, Dominique

(56) Documents cités:
- EP-A- 0 925 806
- US-A- 5 501 702
- US-A- 5 522 860
- US-A- 5 902 325

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

Le pilotage de la stimulation implique l'ajustement permanent de divers paramètres tels que fréquence de stimulation, délai atrioventriculaire (DAV) ou encore délai interventriculaire dans le cas d'une stimulation biventriculaire.

Ces divers paramètres sont ajustés en fonction de signaux délivrés par des capteurs, par exemple de ventilation-minute (MV), qui est un facteur représentatif des besoins métaboliques instantanés du patient.

Ce facteur est, de façon classique, évalué par mesure de bioimpédance transpulmonaire, c'est à dire entre le coeur et le boîtier du stimulateur, situé dans le haut du thorax. Cette valeur est de bioimpédance et mesurée par injection d'une impulsion de courant entre le boîtier et une électrode cardiaque, et recueil d'un potentiel différentiel entre un autre point et le boîtier.

Un autre facteur qu'il est souhaitable de connaître est le débit cardiaque car il peut être intéressant, tout particulièrement avec un stimulateur multisite, d'obtenir une indication de ce débit et donc de la fraction d'éjection, qui est le paramètre hémodynamique de référence pour optimiser la stimulation sur les différents sites.

Ce débit peut être évalué par mesure de la pression intracardiaque, comme cela est par exemple proposé par le WO-A-99/34863 (Pacesetter AB), mais au prix d'une sonde spécifique incorporant un capteur piézoélectrique et d'une électronique associée particulière pour exploiter les signaux issus de ce capteur, les convertir et les transmettre au microprocesseur du stimulateur.

Un autre paramètre corrélé au débit cardiaque est l'impédance transvalvulaire, généralement mesurée sur le coeur droit, comme cela est par exemple proposé par le US-A-5 154 171 (Chirife). Ce document propose d'effectuer la mesure de bioimpédance par injection d'une impulsion de courant entre un site ventriculaire et un site auriculaire et recueil d'un potentiel différentiel entre ces deux mêmes points. En pratique, on constate cependant que cette configuration (configuration bipolaire) d'injection/recueil se révèle sensible au mouvement des sondes et ne permet pas une mesure fiable et précise de l'impédance. De plus, cette technique nécessite une électronique particulière pour injecter et recueillir les signaux de mesure, les convertir et les transmettre pour exploitation au microprocesseur du stimulateur.

Le US-A-5 501 702 (Plicchi) propose une configuration réutilisant les circuits préexistants de mesure de la ventilation-minute, par injection/recueil intracardiaques et non plus injection/recueil transpulmonaire, de manière à mesurer des valeurs de bioimpédance intracardiaques corrélées à des paramètres hémodynamiques tels que débit cardiaque et fraction d'éjection.

Mais ce dispositif connu met en oeuvre des commutations complexes et multiples, où notamment cathode et anode sont reliés à des transistors de commutation pour assurer les liaisons électriques selon le cas au circuit de mesure, aux électrodes atriales et aux électrodes ventriculaires. En fait, ces commutations multiples entraînent une complexification telle du système que celui-ci est en pratique irréalisable.

L'un des buts de l'invention est de proposer une autre configuration permettant d'aboutir au même résultat - c'est-à-dire la réutilisation de la chaîne de mesure de ventilation-minute pour évaluer des impédances intracardiaques - mais avec une économie de moyens bien supérieure rendant le système simple et avantageux à réaliser.

Plus précisément, l'invention concerne un dispositif du type décrit dans le US-A-5 501 702 précité correspondant au préambule de la revendication 1, et comprenant les moyens particuliers énoncés dans la partie caractérisante de cette revendication 1. Les sous-revendications visent des formes de réalisation subsidiaires avantageuses.

On va maintenant décrire diverses mises en oeuvres de l'invention, en référence aux dessins annexés, sur lesquels les mêmes références numériques et littérales désignent des éléments semblables sur les différentes figures.

La figure 1 illustre une configuration connue de mesure de la ventilation-minute depuis l'oreillette.

Les figures 2 et 3 montrent des modifications de la configuration de la figure 1 pour la mesure de l'impédance auriculo-ventriculaire et de l'impédance transvalvulaire, respectivement.

La figure 4 illustre une configuration connue de mesure de la ventilation-minute depuis le ventricule.

Les figures 5 et 6 montrent des modifications de la configuration de la figure 4 pour la mesure de l'impédance interventriculaire et de l'impédance transvalvulaire, respectivement.

La figure 7 représente schématiquement les transistors de commutation des sites permettant de réaliser par une programmation appropriée les différentes configurations des figures 1 à 6.

Les figures 8 et 9 sont des représentations simplifiées de la configuration par commutation de masses opérée selon la figure 7.

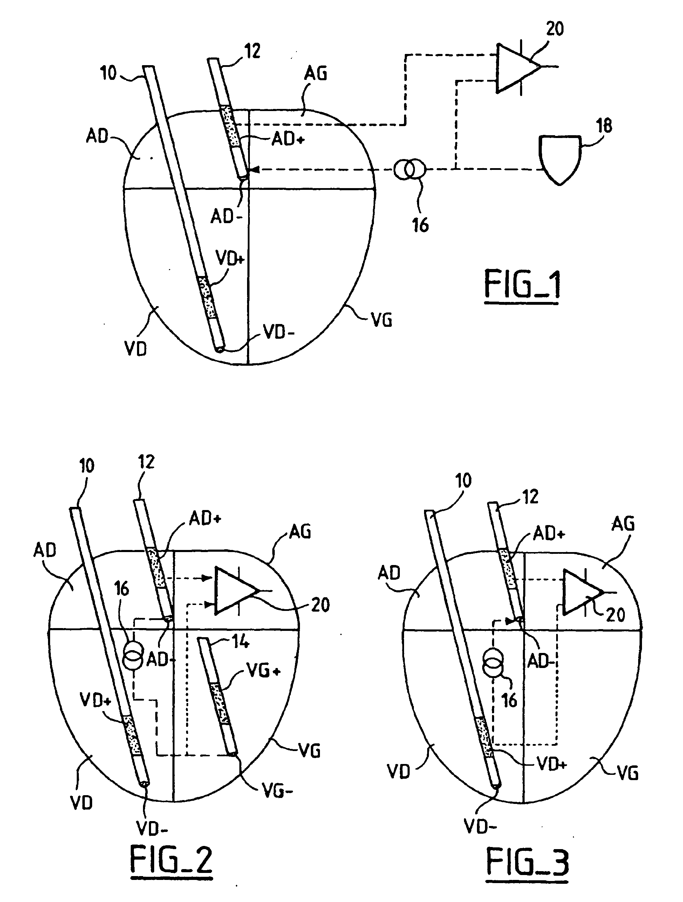

La figure 1 représente schématiquement un muscle cardiaque avec ses quatre cavités : oreillette droite AD, oreillette gauche AG, ventricule droit VD et ventricule gauche VG.

Une sonde ventriculaire 10 est introduite dans le ventricule droit VD, avec une électrode proximale annulaire référencée VD+ et une électrode distale d'extrémité référencée VD-.

Une sonde auriculaire 12 est introduite dans l'oreillette droite AD, avec une électrode proximale annulaire référencée AD+ et une électrode distale d'extrémité référencée AD-.

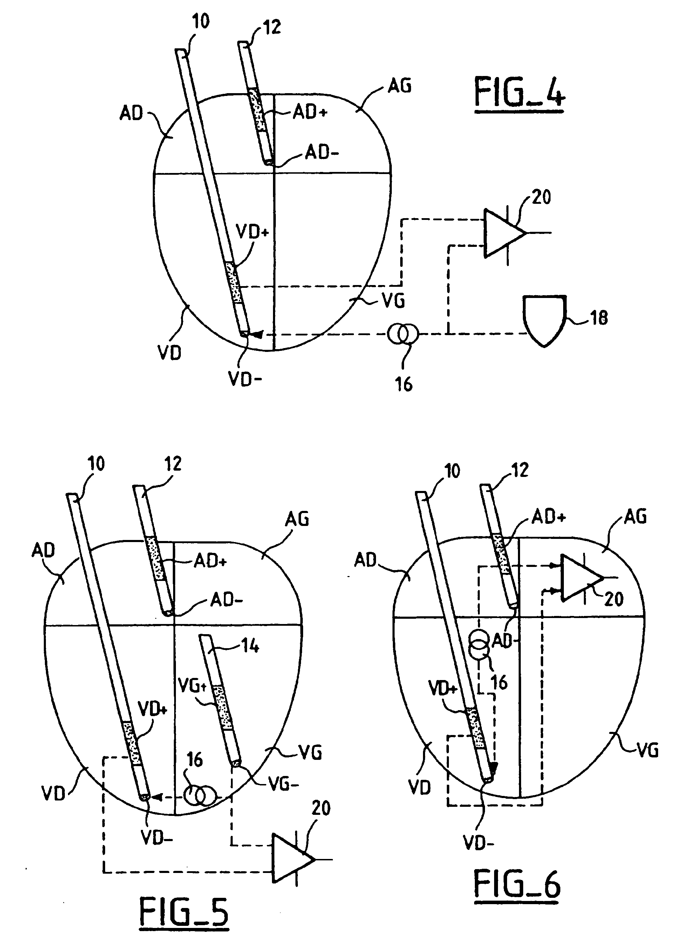

Le cas échéant, il peut également être prévu (comme sur les figures 2 et 5) une sonde 14 sur le ventricule gauche VG, par exemple pour permettre une stimulation biventriculaire (configuration triple chambre) et/ou une sonde sur l'oreillette gauche AG, si l'on souhaite réaliser un recueil de signaux et/ou une stimulation sur les deux oreillettes (configuration quadruple chambre).

Les électrodes des sondes sont reliées à un boîtier comprenant divers circuits de détection, de stimulation et de commande, par exemple un boîtier de stimulateur multisite tel que celui décrit dans le EP-A-0 925 806 (ELA Médical), auquel on pourra se référer pour de plus amples détails. On notera que le dispositif décrit dans ce document comporte des moyens de commutation permettant de relier sélectivement et selon diverses configurations possibles les différents étages du stimulateur de façon variable et évolutive. La commutation selon les différentes configurations voulues y est opérée par une logique câblée et/ou une programmation appropriée du microprocesseur assurant le pilotage de transistors MOS.

La configuration (connue) illustrée figure 1 permet la mesure de la ventilation-minute depuis l'oreillette.

Cette mesure se fait par injection d'une impulsion de courant, schématisée par le générateur de courant 16, entre un premier site auriculaire (dans l'exemple illustré, l'électrode distale auriculaire AD-) et le boîtier métallique 18 du dispositif. Le courant injecté est par exemple un courant de 320 µA délivré sous la forme d'une impulsion de largeur 5 µs. Le potentiel différentiel engendré par cette impulsion de courant est recueilli et mesuré, comme cela est schématisé par l'amplificateur opérationnel 20, entre un site auriculaire (ici, l'électrode proximale auriculaire AD+) distinct de celui utilisé (AD-) pour l'injection, d'une part, et le boîtier 18, d'autre part. Le signal ainsi recueilli donne une indication des besoins métaboliques instantanés du patient.

On notera que cette configuration de mesure de la ventilation-minute est une configuration tripolaire, avec un point (le boîtier) commun à l'injection et au recueil, point qui donne donc ici le potentiel de référence pour la mesure.

La chaîne de mesure que l'on vient de décrire, initialement destinée à une mesure de la ventilation-minute (impédance transpulmonaire), peut être utilisée pour mesurer des impédances intracardiaques.

À cet effet, la même configuration tripolaire (un point commun formant référence de potentiel, un point d'injection et un point de recueil) est conservée, en changeant simplement le site donnant la référence de potentiel. Cette modification peut se faire simplement par une commutation interne au dispositif multisite, de la manière exposée dans le EP-A-0 925 806 précité.

Dans le cas de la figure 2, la liaison au boîtier 18 est remplacée par une liaison à une électrode ventriculaire gauche, ici l'électrode distale ventriculaire gauche VG-. En d'autres termes, l'électrode distale ventriculaire gauche VG- devient la référence de potentiel en lieu et place du boîtier 18 de la figure 1. Les sites d'injection (électrode distale auriculaire AD-) et de recueil (proximale auriculaire AD+), quant à eux, ne sont pas modifiés.

Cette configuration permet de mesurer l'impédance auriculo-ventriculaire, qui est représentative du débit cardiaque et peut notamment être utilisée pour ajuster la fréquence cardiaque, le délai atrio-ventriculaire, ou le délai inter-ventriculaire dans le cas d'une stimulation biventriculaire.

Le courant injecté pour la mesure de cette impédance auriculo-ventriculaire est par exemple un courant de 40 µA délivré sous la forme d'une impulsion de largeur 5 µs.

Par ailleurs, le recueil est opéré dans des bandes de fréquences différentes : basse fréquence pour la mesure de la ventilation-minute, fréquence plus élevée pour la mesure de l'impédance auriculo-ventriculaire ; cette modification de la bande de fréquences peut résulter d'un déplacement des points de coupure du filtre, modification très aisée à réaliser par des moyens logiciels si le filtrage est un filtrage numérique.

On notera incidemment qu'il n'est pas indispensable que les sites ventriculaire et auriculaire se situent du même côté du coeur. Ainsi, dans le cas de la figure 2 décrite ci-dessus, il est possible de ne pas avoir de sonde ventriculaire droite, la configuration choisie (éventuellement, programmée) étant alors une configuration oreillette droite/ventricule gauche.

Dans la variante de configuration de la figure 3, le potentiel de référence choisi est celui d'une électrode ventriculaire droite, en l'espèce l'électrode proximale ventriculaire droite VD+. En d'autres termes, par rapport à la configuration connue de la figure 1, dans la configuration de la figure 3 la liaison au boîtier 18 formant le point commun de la configuration tripolaire est remplacée par une liaison à l'électrode proximale ventriculaire droite VD+.

Cette configuration permet d'obtenir une mesure de l'impédance transvalvulaire, paramètre représentatif de la fraction d'éjection.

La figure 4 illustre une autre configuration (connue) de mesure de la ventilation-minute. La différence par rapport à la configuration de la figure 1 tient au fait que les points d'injection et de recueil sont situés dans le ventricule droit au lieu de l'être dans l'oreillette droite : l'injection se fait alors entre le boîtier 18 et l'électrode distale ventriculaire droite VD-, et le recueil entre le boîtier 18 et l'électrode proximale ventriculaire droite VD+. Dans cette configuration comme celle de la figure 1, la référence de potentiel (point commun de la configuration tripolaire) est toujours le boîtier 18.

La configuration de la figure 5 est la même que celle de la figure 4, à cette différence près que le potentiel de référence n'est plus le boîtier 18 mais une électrode ventriculaire gauche, en l'espèce l'électrode distale ventriculaire gauche VG-.

Cette configuration permet notamment une mesure de l'impédance inter-ventriculaire, qui est un paramètre important pour piloter le délai inter-ventriculaire dans le cas d'une stimulation biventriculaire.

Dans la variante de la figure 6, on choisit comme référence de potentiel, en lieu et place du boîtier 18 de la configuration de la figure 4, une électrode auriculaire droite, en l'espèce l'électrode proximale auriculaire droite AD+.

Cette configuration permet d'obtenir une mesure de l'impédance transvalvulaire, comme dans le cas de la figure 3, mais cette fois-ci à partir du ventricule et non plus de l'oreillette.

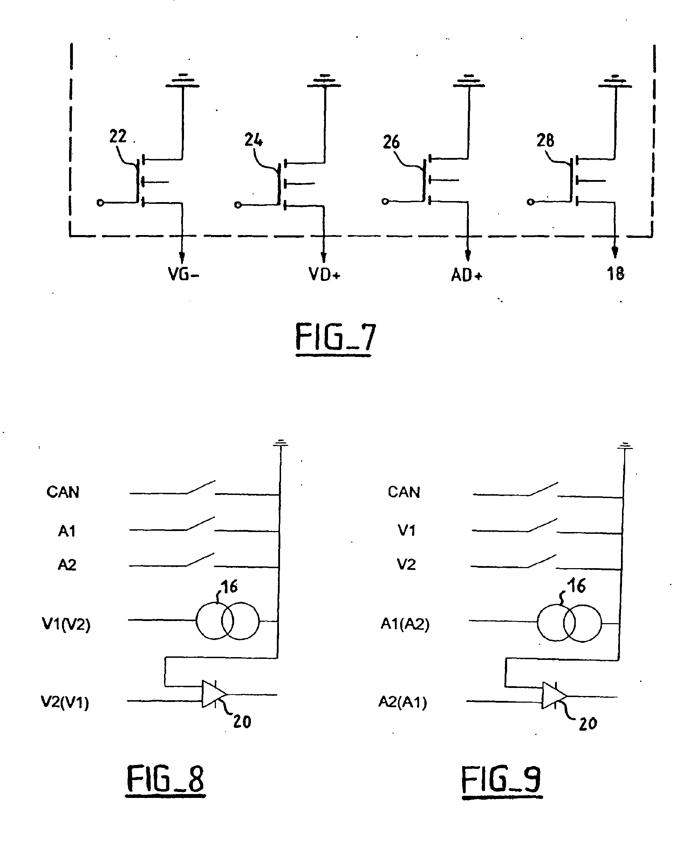

La figure 7 représente schématiquement des transistors de commutation permettant de réaliser par une programmation appropriée les différentes configurations des figures 1 à 6 ci-dessus, simplement par une mise à la masse de certains sites. Ainsi :
- l'activation du transistor 22 met à la masse l'électrode VG-,
- l'activation du transistor 24 met à la masse l'électrode VD+,
- l'activation du transistor 26 met à la masse l'électrode AD+,
- l'activation du transistor 28 met à la masse le boîtier 18.

La sélection des diverses configurations peut alors être opérée de la manière suivante par simple pilotage des transistors, sous le contrôle par exemple d'un microprocesseur ou d'une logique câblée :
- configuration de la figure 1 : le transistor 28 est activé et durant l'injection la masse électrique est ramenée sur le boîtier ;
- configuration de la figure 2 : le transistor 22 est activé et durant l'injection la masse électrique est ramenée sur VG- ;
- configuration de la figure 3 : le transistor 24 est activé et durant l'injection la masse électrique est ramenée sur VD+ ;
   (pour ces trois configurations, il y a injection sur AD- et recueil sur AD+)
- configuration de la figure 4 : le transistor 28 est activé et durant l'injection la masse électrique est ramenée sur le boîtier ;
- configuration de la figure 5 : le transistor 22 est activé et durant l'injection la masse électrique est ramenée sur VG- ;
- configuration de la figure 6, le transistor 26 est activé et durant l'injection la masse électrique est ramenée sur AD+ ;
   (pour ces trois configurations, il y a injection sur VD- et recueil sur VD+)

On notera que, pour toutes les configurations décrites plus haut, il est possible d'inverser le rôle des électrodes proximales et des électrodes distales.

Par ailleurs, l'oreillette gauche peut également être utilisée à la place du ventricule gauche, bien que ce choix entraîne une plus grande complexité et de moindres performances, du fait notamment du recours obligé à des sondes coronaires.

La figure 8 illustre de façon schématique la configuration selon l'invention, fonctionnant par commutation de masses. Comme on peut le voir sur cette figure, les branches provenant des électrodes ventriculaires (V1 et V2) et aboutissant aux circuits d'injection de courant 16 et de mesure 20 ne sont pas commutées ; seules sont commutées, selon que l'on veut mesurer la ventilation-minute ou bien l'impédance, les branches provenant du boîtier (convertisseur analogique-numérique CAN) et de chacune des électrodes auriculaires (A1 et A2) - et cette commutation est opérée très simplement par mise à la masse ou isolement de la masse.

La figure 9 est homologue de la figure 8, les rôles des oreillettes et des ventricules ayant seulement été inversés.

## Revendications

1. Un dispositif médical implantable actif, notamment un stimulateur cardiaque, défibrillateur et/ou cardioverteur, du type multisite le dispositif comprenant : électrodes capables d'être placées en une pluralité de sites respectifs distincts comportant au moins un site ventriculaire et un site auriculaire, un circuit de recueil de signaux cardiaques pour détecter un potentiel de dépolarisation avec lequel les électrodes sont reliées, un circuit de stimulation, pour appliquer des impulsions de stimulation sur au moins certains desdits sites,
* des moyens d'évaluation des besoins métaboliques du patient par mesure de bioimpédance transpulmonaire, ces moyens comprenant :
■ un circuit d'injection de courant (16) entre une première sortie reliée au boîtier (18) du dispositif et une seconde sortie reliée à un point d'injection situé en un premier site atrial (AD-), ou ventriculaire (VD-), et
un circuit de mesure du potentiel différentiel (20) engendré par l'injection de courant entre une première entrée reliée au boîtier (18) du dispositif et une seconde entrée reliée à un point de mesure situé en un second site atrial (AD+), ou ventriculaire (VD+) respectivement,
* des moyens de mesure de bioimpédance intracardiaque, comprenant des moyens de commutation aptes à :
■ isoler du boîtier (18) la première sortie du circuit d'injection de courant (16) et la première entrée du circuit de mesure de potentiel différentiel (20), et
■ relier la première sortie du circuit d'injection de courant et la première entrée du circuit de mesure de potentiel différentiel à un site commun de référence de potentiel, atrial (AD+, fig. 6) ou ventriculaire (VG-, fig. 2 ; VD+, fig. 3 ; VG-, fig. 5), distinct des sites (VD-,VD+, fig. 6 ; AD-,AD+, fig. 2 ; AD-,AD+, fig. 3 ; VD-,VD+, fig. 5) auxquels sont reliés la seconde sortie du circuit d'injection de courant et la seconde entrée du circuit de mesure de potentiel différentiel,
de manière à permettre une mesure d'impédance intracardiaque à partir du signal délivré par le circuit de mesure de potentiel différentiel, dispositif **caractérisé en ce que** lesdits moyens de commutation sont des moyens (22, 24, 26, 28) de commutation de masses électriques, opérant par mise à la masse, ou isolement de la masse, des branches provenant du boîtier (CAN) et des électrodes auriculaires, respectivement ventriculaires (A₁, A₂, V₁, V₂), tandis que les branches provenant des électrodes ventriculaires, respectivement auriculaires (V₁, V₂, A₁, A₂), et aboutissant au circuit d'injection de courant (16) au circuit de mesure de potentiel différentiel (20) ne sont pas commutées.

2. Le dispositif de la revendication 1, dans lequel le circuit de mesure comprend des moyens de filtrage dont les fréquences de coupure sont déplaçables par les moyens de commutation dans le sens d'une élévation de la bande de fréquences mesurée.

3. Le dispositif de la revendication 1, dans lequel lesdits moyens de commutation sont des moyens aptes à :
- relier la première sortie du circuit d'injection de courant et la première entrée du circuit de mesure de potentiel différentiel à un site ventriculaire gauche (VG-) formant ledit site commun de référence de potentiel, et
- relier la seconde sortie du circuit d'injection de courant et la seconde entrée du circuit de mesure de potentiel différentiel à des sites auriculaires respectifs distincts (AD-,AD+),
ladite bioimpédance intracardiaque mesurée étant une bioimpédance auriculo-ventrieulaire.

4. Le dispositif de la revendication 1, dans lequel lesdits moyens de commutation sont des moyens aptes à :
- relier la première sortie du circuit d'injection de courant et la première entrée du circuit de mesure de potentiel différentiel à un site ventriculaire droit (VD+) formant ledit site commun de référence de potentiel, et
- relier la seconde sortie du circuit d'injection de courant et la seconde entrée du circuit de mesure de potentiel différentiel à des sites auriculaires respectifs distincts (AD-,AD+),
ladite bioimpédance intracardiaque mesurée étant une bioimpédance transvalvulaire.

5. Le dispositif de la revendication 1, dans lequel lesdits moyens de commutation sont des moyens aptes à :
- relier la première sortie du circuit d'injection de courant et la première entrée du circuit de mesure de potentiel différentiel à un site ventriculaire gauche (VG-) formant ledit site commun de référence de potentiel, et
- relier la seconde sortie du circuit d'injection de courant et la seconde entrée du circuit de mesure de potentiel différentiel à des sites ventriculaires droits respectifs distincts (VD-,VD+),
ladite bioimpédance intracardiaque mesurée étant une bioimpédance interventriculaire.

6. Le dispositif de la revendication 1, dans lequel lesdits moyens de commutation sont des moyens aptes à :
- relier la première sortie du circuit d'injection de courant et la première entrée du circuit de mesure de potentiel différentiel à un site auriculaire droit (AD+) formant ledit site commun de référence de potentiel, et
- relier la seconde sortie du circuit d'injection de courant et la seconde entrée du circuit de mesure de potentiel différentiel à des sites ventriculaires droits respectifs distincts (VD-,VD+),
ladite bioimpédance intracardiaque mesurée étant une bioimpédance transvalvulaire.

7. Le dispositif de la revendication 1, dans lequel lesdits premier et second sites auriculaires, ou ventriculaires, sont définis par une électrode proximale et une électrode distale d'une même sonde auriculaire, ou ventriculaire respectivement.

## Claims

1. An active implantable medical device, in particular a pacemaker, defibrillator and/or cardioverter, of the multisite type, the device comprising: electrodes adapted to be placed in a plurality of respective distinct sites comprising at least one ventricular site and one atrial site, a circuit for collecting cardiac signals to detect a depolarization potential to which the electrodes are connected, a stimulation circuit for applying stimulation pulses to at least certain of said sites, means for evaluating the patient's metabolic needs by measuring a transpulmonary bio-impedance, these means comprising:
■ a circuit (16) for injecting a current between a first output connected to the device's casing (18) and a second output connected to an injection point located in a first atrial (AD-) or ventricular (VD-) site, and
■ a circuit (20) for measuring the differential potential generated by the current injection between a first input connected to the device's casing (18) and a second input connected to a measurement point located in a second atrial (AD+) or ventricular (VD+) site, respectively,
means for measuring an intracardiac bio-impedance, comprising switching means able to:
■ isolate from the casing (18) the first output of the current injection circuit (16) and the first input of the differential potential measuring circuit (20), and
■ connect the first output of the current injection circuit and the first input of the differential potential measuring circuit to a common atrial (AD+, fig. 6) or ventricular (VG-, fig. 2; VD+, fig. 3; VG-, fig.5) reference potential site distinct from the sites (VD-, VD+, fig. 6; AD-, AD+, fig. 2; AD-, AD+, fig. 3; VD-, VD+, fig. 5) to which are connected the second output of the current injection circuit and the second input of the differential potential measuring circuit,
in order to allow a measurement of an intracardiac impedance from the signal delivered by the differential potential measuring circuit, a device, **characterized in that** said switching means are means (22, 24, 26, 28) for switching electric grounds operating by grounding or isolating from ground the branches coming from the casing (ADC) and the atrial, respectively ventricular electrodes (A1, A2; V1, V2), while the branches coming from the ventricular, respectively atrial electrodes (V1, V2; A1, A2) and ending at the current injection circuit (16) and the differential potential measuring circuit (20) are not switched.

2. The device of claim 1, wherein the measuring circuit comprises filtering means, the cut off frequencies of which are movable by the switching means in the direction of an increase of the measured frequency band.

3. The device of claim 1, wherein said switching means are means able to:
- connect the first output of the current injection circuit and the first input of the differential potential measuring circuit to a left ventricular site (VG-) forming said common reference potential site, and
- connect the second output of the current injection circuit and the second input of the differential potential measuring circuit to distinct respective atrial sites (AD-, AD+), said measured intracardiac bio-impedance being an atrioventricular bio-impedance.

4. The device of claim 1, wherein said switching means are means able to:
- connect the first output of the current injection circuit and the first input of the differential potential measuring circuit to a right ventricular site (VD+) forming said common reference potential site, and
- connect the second output of the current injection circuit and the second input of the differential potential measuring circuit to distinct respective atrial sites (AD-, AD+), said measured intracardiac bio-impedance being a transvalvular bio-impedance.

5. The device of claim 1, wherein said switching means are means able to:
- connect the first output of the current injection circuit and the first input of the differential potential measuring circuit to a left ventricular site (VG-) forming said common reference potential site, and
- connect the second output of the current injection circuit and the second input of the differential potential measuring circuit to distinct respective right ventricular sites (VD-, VD+),
said measured intracardiac bio-impedance being an inter-ventricular bio-impedance.

6. The device of claim 1, wherein said switching means are means able to:
- connect the first output of the current injection circuit and the first input of the differential potential measuring circuit to a right atrial site (AD+) forming said common reference potential site, and
- connect the second output of the current injection circuit and the second input of the differential potential measuring circuit to distinct respective right ventricular sites (VD-,VD+), said measured intracardiac bio-impedance being a transvalvular bio-impedance.

7. The device of claim 1, wherein said first and second atrial or ventricular sites are defined by a proximal electrode and a distal electrode of a same atrial or ventricular probe, respectively.

## Patentansprüche

1. Aktive implantierbare medizinische Vorrichtung, insbesondere Herzschrittmacher, Defibrillator und/oder Kardioverter, vom Multisite-Typ, wobei die Vorrichtung folgendes umfasst: Elektroden, die geeignet sind, an einer Mehrzahl von jeweiligen unterschiedlichen Sitzen platziert zu werden, die mindestens einen Herzkammersitz und einen Herzvorhofsitz umfassen, eine Schaltung zur Aufnahme von Herzsignalen zur Erfassung eines Depolarisationspotentials, mit welcher die Elektroden verbunden sind, eine Stimulationsschaltung zur Abgabe von Stimulationsimpulsen an wenigstens bestimmte der Sitze, Mittel zur Bestimmung der Stoffwechselbedürfnisse des Patienten durch Messung der transpulmonalen Bioimpedanz, wobei diese Mittel folgendes umfassen:
■ eine Schaltung (16) zur Stromeinspeisung zwischen einem ersten mit dem Gehäuse (18) der Vorrichtung verbundenen Ausgang und einem zweiten Ausgang, der mit einem Einspeisungspunkt verbunden ist, der sich an einem ersten atrialen (AD-) oder ventrikulären (VD-) Sitz befindet, und
■ eine Schaltung (20) zur Messung der Differenzspannung, die durch die Stromeinspeisung zwischen einem ersten mit dem Gehäuse (18) der Vorrichtung verbundenen Eingang und einem zweiten Eingang, der mit einem Messpunkt verbunden ist, der sich jeweils an einem zweiten atrialen (AD+) oder ventrikulären (VD+) Sitz befindet, erzeugt wird,
Mittel zur Messung einer intrakardialen Bioimpedanz, die Mittel zur Umschaltung umfassen, die geeignet sind:
■ den ersten Ausgang der Stromeinspeisungsschaltung (16) und den ersten Eingang der Schaltung (20) zur Messung einer Differenzspannung vom Gehäuse (18) zu isolieren, und
■ den ersten Ausgang der Stromeinspeisungsschaltung und den ersten Eingang der Schaltung zur Messung einer Differenzspannung mit einem gemeinsamen, atrialen (AD+, Fig. 6) oder ventrikulären (VG-, Fig. 2; VD+, Fig. 3; VG-, Fig. 5) Referenzpotentialsitz zu verbinden, der von den Sitzen (VD-, VD+, Fig. 6; AD-, AD+, Fig. 2; AD-, AD+, Fig. 3; VD-, VD+, Fig. 5) getrennt ist, mit welchen der zweite Ausgang der Stromeinspeisungsschaltung und der zweite Eingang der Schaltung zur Messung einer Differenzspannung verbunden sind,
um eine Messung der intrakardialen Impedanz anhand des durch die Schaltung zur Messung einer Differenzspannung gelieferten Signals zu erlauben, Vorrichtung, die **dadurch gekennzeichnet ist, dass** die Mittel zur Umschaltung Mittel (22, 24, 26, 28) zur Umschaltung von elektrischen Massen sind, die durch das Verbinden mit der Masse oder Isolieren von der Masse der Zweige arbeiten, die vom Gehäuse (ADW) und den Herzvorhof- bzw. Herzkammerelektroden (A1, A2; V1, V2) ausgehen, während die Zweige, welche von den Herzkammer- bzw. Herzvorhofelektroden (V1, V2; A1, A2) ausgehen und in die Stromeinspeisungsschaltung (16) und die Schaltung (20) zur Messung einer Differenzspannung münden, nicht umgeschaltet werden.

2. Vorrichtung nach Anspruch 1, bei welcher die Messschaltung Filtermittel umfasst, deren Grenzfrequenzen durch die Mittel zur Umschaltung in Richtung einer Erhöhung des gemessenen Frequenzbandes versetzbar sind.

3. Vorrichtung nach Anspruch 1, bei welcher die Mittel zur Umschaltung Mittel sind, die geeignet sind:
- den ersten Ausgang der Stromeinspeisungsschaltung und den ersten Eingang der Schaltung zur Messung einer Differenzspannung mit einem linken Herzkammersitz (VG-) zu verbinden, der den gemeinsamen Referenzpotentialsitz bildet, und
- den zweiten Ausgang der Stromeinspeisungsschaltung und den zweiten Eingang der Schaltung zur Messung einer Differenzspannung mit jeweiligen unterschiedlichen Herzvorhofsitzen (AD-, AD+) zu verbinden,
wobei die gemessene intrakardiale Bioimpedanz eine atrioventrikuläre Bioimpedanz ist.

4. Vorrichtung nach Anspruch 1, bei welcher die Mittel zur Umschaltung Mittel sind, die geeignet sind:
- den ersten Ausgang der Stromeinspeisungsschaltung und den ersten Eingang der Schaltung zur Messung einer Differenzspannung mit einem rechten Herzkammersitz (VD+) zu verbinden, der den gemeinsamen Referenzpotentialsitz bildet, und
- den zweiten Ausgang der Stromeinspeisungsschaltung und den zweiten Eingang der Schaltung zur Messung einer Differenzspannung mit jeweiligen unterschiedlichen Herzvorhofsitzen (AD-, AD+) zu verbinden,
wobei die gemessene intrakardiale Bioimpedanz eine transvalvuläre Bioimpedanz ist.

5. Vorrichtung nach Anspruch 1, bei welcher die Mittel zur Umschaltung Mittel sind, die geeignet sind:
- den ersten Ausgang der Stromeinspeisungsschaltung und den ersten Eingang der Schaltung zur Messung einer Differenzspannung mit einem linken Herzkammersitz (VG-) zu verbinden, der den gemeinsamen Referenzpotentialsitz bildet, und
- den zweiten Ausgang der Stromeinspeisungsschaltung und den zweiten Eingang der Schaltung zur Messung einer Differenzspannung mit jeweiligen unterschiedlichen rechten Herzkammersitzen (VD-, VD+) zu verbinden,
wobei die gemessene intrakardiale Bioimpedanz eine interventrikuläre Bioimpedanz ist.

6. Vorrichtung nach Anspruch 1, bei welcher die Mittel zur Umschaltung Mittel sind, die geeignet sind:
- den ersten Ausgang der Stromeinspeisungsschaltung und den ersten Eingang der Schaltung zur Messung einer Differenzspannung mit einem rechten Herzvorhofsitz (AD+) zu verbinden, der den gemeinsamen Referenzpotentialsitz bildet, und
- den zweiten Ausgang der Stromeinspeisungsschaltung und den zweiten Eingang der Schaltung zur Messung einer Differenzspannung mit jeweiligen unterschiedlichen rechten Herzkammersitzen (VD-, VD+) zu verbinden,
wobei die gemessene intrakardiale Bioimpedanz eine transvalvuläre Bioimpedanz ist.

7. Vorrichtung nach Anspruch 1, bei welcher der erste und zweite Herzvorhof- oder Herzkammersitz jeweils durch eine nahe gelegene Elektrode und eine entfernte Elektrode einer selben Herzvorhof- oder Herzkammersonde definiert sind.
